# EUROPÄISCHE PATENTSCHRIFT

(11) **EP 1 704 223 B1**
(45) Veröffentlichungstag und Bekanntmachung des Hinweises auf die Patenterteilung: **11.11.2009**
(21) Anmeldenummer: 05701501.8
(22) Anmeldetag: 12.01.2005
(51) Int. Cl.: C12M 1/26, G01N 1/20, G01N 1/10

(54) **VORRICHTUNG UND VERFAHREN ZUR PROBENAHME**
DEVICE AND METHOD FOR TAKING SAMPLES
DISPOSITIF ET PROCEDE DE PRELEVEMENT D'ECHANTILLONS

(30) Priorität: 14.01.2004 DE 102004001916
(43) Veröffentlichungstag der Anmeldung: 27.09.2006
(73) Patentinhaber: MPG Max-Planck-Gesellschaft zur Förderung der Wissenschaften e.V., 80539 München (DE)
(72) Erfinder: SANN, Heiner, 39104 Magdeburg (DE); FRANZ, Detlef, 06217 Merseburg (DE); BOCK, Andreas, 39114 Magdeburg (DE); STOLL, Klaus Dieter, 39179 Barleben (DE); REICHL, Udo, 30938 Burgwedel (DE)
(74) Vertreter: Hannke, Christian
(86) Internationale Anmeldenummer: PCT/EP2005/050115
(87) Internationale Veröffentlichungsnummer: WO 2005/068606

(56) Entgegenhaltungen:
- DE-A1- 4 407 439
- US-A- 4 046 011
- US-A1- 2001 004 449
- PATENT ABSTRACTS OF JAPAN Bd. 010, Nr. 017 (C-324), 23. Januar 1986 (1986-01-23) & JP 60 172279 A (HITACHI SEISAKUSHO KK), 5. September 1985 (1985-09-05)
- PATENT ABSTRACTS OF JAPAN Bd. 017, Nr. 589 (C-1124), 27. Oktober 1993 (1993-10-27) & JP 05 176752 A (TOYO ENG CORP), 20. Juli 1993 (1993-07-20)

## Beschreibung

Die Erfindung betrifft eine Vorrichtung und ein Verfahren zur Probenahme von flüssigen Proben aus mit einem Medium gefüllten Behältern und/oder Rohren, insbesondere aus Fermentern, mittels Unterdruck, wobei innerhalb einer Probesonde ein als Rückstellventil wirkendes Element als Eingang für die flüssige Probe angeordnet ist, gemäß den Oberbegriffen der Patentansprüche 1 und 14.

Eine Vorrichtung zur Entnahme von Proben aus einem Fermenter ist aus der DE 32 49 399 T1 und der US 4 689 306 bekannt. Für die Entnahme der Probe weist die Vorrichtung einen Probenehmer in Form eines normal geschlossenen Ventils auf, an dessen Gehäuse auf der Seite eines Kontaktes mit einem Probeaufnehmer ein ringförmiger Vorsprung ausgebildet ist, der zur Aufnahme einer Ringnut eines Gehäuses eines normal geschlossenen Ventils des Probeaufnehmers vorgesehen ist. Zwischen dem normal geschlossenen Ventil des Probeaufnehmers und einem Halt des Probeaufnehmers ist ein bakteriendichter Filter angeordnet, der zu dem normal geschlossenen Ventil des Probenehmers beabstandet ist. Diese Beabstandung hat zur Folge, dass bei der Entnahme einer Probe ein dadurch entstandener Zwischenraum innerhalb der Vorrichtung ebenso mit der Probeflüssigkeit gefüllt werden muss, jedoch nicht als zu überprüfende Probeflüssigkeit in den Probeaufnehmer mitaufgenommen wird. Ein dadurch entstandenes Totvolumen der entnommenen Probe bewirkt, dass zum einen die Probenahme nicht sparsam im Bezug auf das benötigte Probevolumen pro Probeentnahme durchgeführt werden kann und zum anderen ein Restvolumen aus der vorangegangenen Probeentnahme innerhalb der Vorrichtung zurückgeblieben ist, das aufgrund seiner Eigenschaften zu einer Verfälschung der Prüfergebnisse der nachfolgend durchgeführten Probeentnahme mit gewünschter Sterilität führen kann.

US 4,046,011 beschreibt beispielsweise ein Einwege-Ventil einer Vorrichtung zur Entnahme flüssiger Proben, welches in einem guasi-offenen System unter Atmosphärendruck arbeitet, wobei zum Öffnen bzw. Schließen eines Diaphragmas mittels einer druckführbaren Zuführleitung ein Überdruck erzeugt wird.

Eine weitere Vorrichtung zur Probennahme wird in US 2001/0004449 A1 offenbart, wobei eine innenliegende Probenröhre mit einer sie umgebenden Röhre zur Versorgung mit Reinigungsflüssigkeit über eine am Ort der Probennahme gelegenen Dichtung verbunden ist. Die gegenseitige Abdichtung der Röhren bei Probenahme bzw. Spülung erfolgt über eine Auf- bzw. Abwärtsbewegung der beiden Röhren.

JP 60 172279 A zeigt eine zur Probenahme geeignete Kolbeneinrichtung, welche die zu entnehmende Flüssigkeit mittels Überdruck durch ein Ventil sowie eine Abführleitung aus der Vorrichtung verdrängt.

Derartige Probenahmevorrichtungen weisen ein Totvolumen innerhalb des Totraumes bei der Probenahme auf, das zum Einen eine Probenahme mit höherem Volumen erfordert und zum Anderen die Gefahr einer Vermischung der in dem Totvolumen angeordneten Restprobe aus der vorangegangenen Probenahme mit den Probesubstanzen der neuen Probenahme aufwirft.

Zudem lassen derartige Vorrichtungen aufgrund des begrenzt ausgebildeten Zwischenraumes Probenahmen mit festgelegtem Volumen zu. Die Entnahme von Proben mit größeren Volumen würde ein vollständiges Auswechseln der Probenahmevorrichtung erfordern.

Des Weiteren ermöglichen derartige Probenahmevorrichtungen lediglich eine bestimmte Anzahl von zu entnehmenden Proben pro Zeiteinheit, da hierfür zeitraubende Handgriffe erforderlich sind. Die überwiegende Anzahl der herkömmlichen Probenahmevorrichtungen erfordern den Einbau eines Filters/einer Filtermembran, der/die die Sterilität der entnommenen Probe sicherstellen soll. Dies hat eine aufwendige Zusatzkonstruktion der Vorrichtungen zur Folge.

Demzufolge liegt der vorliegenden Erfindung die Aufgabe zugrunde, eine Vorrichtung und ein Verfahren zur Probenahme von flüssigen oder gasförmigen Proben aus mit einem Medium gefüllten Behälter und/oder Rohren mittels Unterdruck zur Verfügung zu stellen, bei der/dem eine Entnahme der Probe ohne Totvolumen unter zuverlässigen Bedingungen möglich ist, wobei die Vorrichtung einen einfachen Aufbau aufweist und kostengünstig in ihrer Herstellung ist.

Diese Aufgabe wird vorrichtungsseitig durch die Merkmale des Patentanspruches 1 und verfahrensseitig durch die Merkmale des Patentanspruches 14 gelöst.

Ein wesentlicher Punkt der Erfindung liegt darin, dass bei einer Vorrichtung zur Entnahme von flüssigen oder gasförmigen Proben aus mit einem Medium gefüllten ersten Behältern und/oder Rohren, insbesondere aus Fermentern, mittels Unterdruck eine gasführbare Zuführleitung und eine probeabführbare Abführleitung derart angeordnet sind, dass sie auf der dem im ersten Behälter angeordneten Medium abgewandten Seite eines als Rückstellventil wirkenden Elementes liegen. Das als Rückstellventil wirkende Element bildet einen Eingang für die flüssige oder gasförmige Probe und kann innerhalb eines probeaufnehmenden zweiten Behälters oder in einer Leitungsanordnung zwischen Zu- und Abführleitung angeordnet sein. Auf diese Weise wird eine einfache und kostengünstige Vorrichtung zur sterilen Probenahme zur Verfügung gestellt, indem mittels Druckdifferenz zwischen dem ersten Behälter und den Leitungen der Vorrichtung, beispielsweise innerhalb des zweiten Behälters, ein selbsttätiges sich Öffnen und sich Schließen des als Rückstellventil wirkenden Elementes bewirkt wird. Denn zunächst wird mittels eines in den Leitungen und gegebenenfalls in dem zweiten Behälter erzeugten Unterdrucks das als Rückstellventil wirkende Element geöffnet, woraufhin die zu entnehmende Probemenge in die Abführleitung und gegebenenfalls zusätzlich in den zweiten Behälter einströmt. Anschließen wird mittels eines durch ein zugeführtes Gas erzeugten Überdrucks innerhalb der Leitungsanordnung und gegebenenfalls innerhalb des zweiten Behälters ein selbsttätiges Sichschließen des als Rückstellventil wirkenden Elementes bewirkt. Zeitgleich und anschließend findet das vollständige Abführen der zu entnehmenden Probe aus den Leitungen und gegebenenfalls aus dem zweiten Behälter über die Abführleitung mit Hilfe des von dem Gas herrührenden Überdrucks statt. Da der zweite Behälter während des Abführens des Gases vollständig von dem restlichen Medium isoliert ist, ist eine totvolumenfreie Probeentnahme möglich. Ebenso kann bei der Anordnung des Elements innerhalb der Leitungsanordnung ohne das Bestehen eines zweiten Behälters eine totvolumenfreie Probeentnahme erreicht werden, da eine Isolierung der Leitungsanordnung von dem restlichen Medium während des Abführvorganges vorliegt.

Ein derartiges Abführen der Probe mit einem vorbestimmten Probevolumen, welches zum Beispiel mittels einer Pumpe reguliert wird, aus dem Leitungssystem und gegebenenfalls dem zweiten Behälter der Probenahmevorrichtung hat zeitgleich ein zuverlässiges Entleeren des Leitungssystems mittels des zugeführten Gases zur Folge. Da auf diese Weise kein Totraum innerhalb der Probenahmevorrichtung für die Bildung eines Totvolumens entstehen kann, ist eine Entnahme der Probe mit dem gewünschten zu überprüfenden Probevolumen selbst bei kleinen Volumenmengen möglich, ohne dass zusätzliche Probevolumen aufgrund der Bildung eines Totvolumens notwendig wären.

Da das Leitungssystem nach jeder Probeentnahme vollständig mittels des Gases, welches beispielsweise Druckluft sein kann, entleert wird, ist die gesamte Probenahmevorrichtung einschließlich des zweiten Behälters vor Beginn eines weiteren Probenahmevorganges in sich steril. Somit ist eine Probenahme mit einem hohen Grad an Sterilität sichergestellt.

Da eine automatische Entleerung auf einfache und wirkungsvolle Weise im Anschluss an die Probenahme stattfindet, ist die erfindungsgemäße Probenahmevorrichtung als Probenahmemodul automatisiert einsetzbar und ermöglicht den Anschluss von Detektionssystemen, die für eine prozessnahe Kontrolle und Regelung/Steuerung der entnommenen Probe und erfindungsgemäßen Vorrichtung dienen.

Zudem kann der Zeitraum für einen Probenahmevorgang durch geeignete Kombination von dem zu entnehmenden Probenvolumen, der durch die Pumpe bedingten Fördergeschwindigkeit und der benötigten Zeitspanne für die Entleerung des Leitungssystems ausreichend kurz für Online-Messungen gehalten werden. Dies hat eine höhere Anzahl von Probenahmen pro Zeiteinheit zur Folge.

Das Element ist erfindungsgemäß als Rückschlagventil ausgebildet und bildet aufgrund seiner Anordnung eine Übergangsstelle zwischen dem im Fermenter angeordneten Medium und den rückseitig angeordneten Zuführ- und Abführleitungen und ermöglicht somit an dieser Grenzflächenposition ein zuverlässiges und schnelles Trennen der entnommenen Probe von dem restlichen Medium durch einen Schließvorgang.

Ein Integritätstest zur Überprüfung der Dichtigkeit des als Rückstellventil wirkenden Elementes ist zu einem beliebigen Zeitpunkt möglich. Ein derartiger Test kann vor und nach einem Probenahmevorgang durchgeführt werden und ermöglicht demzufolge eine Erhöhung der Betriebssicherheit im Behälter, der einen Bioreaktor darstellen kann, in Verbindung mit der daran befestigten Probenahmevorrichtung und eine Verbesserung der Handhabung der Probenahmevorrichtung.

Für einen zusätzlichen Integritätstest, der die Funktion der Probenahmevorrichtung in situ überprüft, ist die gasführbare Zuführleitung nach einer ersten gasführenden Anschlussleitung zum Verbinden der Zuführleitung mit einem Gasversorgungsanschluss gekoppelt. Eine derartige Kopplung ist ebenso für die Zuführung des Gases zur Entleerung des Leitungssystems erforderlich.

Ein erstes und zweites Ventil sind im Bereich eines ersten und zweiten Endes der ersten gasführenden Anschlussleitung gemäß einer bevorzugten Ausführungsform angeordnet, so dass die Anschlussleitung beiderseitig mittels des Ventils gesperrt werden kann.

Zusätzlich sind ein Drucksensor in der gasführenden Anschlussleitung sowie ein erster Sterilfilter im Bereich des Gasversorgungsanschlusses innerhalb der gasführenden Anschlussleitung angeordnet, so dass der sterile Betrieb des Leitungssystems und insbesondere der gasführenden Anschlussleitung sichergestellt und eine Überprüfung des üblicherweise leichten Überdrucks innerhalb der Gasleitung möglich ist.

Gemäß einer bevorzugten Ausführungsform sind sowohl die Zuführleitung als auch die Abführleitung innerhalb des ersten Behälters angeordnet und weisen zumindest teilweise eine wärmeisolierende Ummantelung auf. Diese Ummantelung kann mit einer Heiz- und/oder Kühleinrichtung zum Temperieren der Zuführ- und Abführleitungen gekoppelt sein, so dass zur Verlängerung der Aussagekraft der entnommenen Probe während des Probeentnahmevorganges eine Temperierung beziehungsweise Kühlung der Probe innerhalb der Probenahmesonde möglich ist.

Die Zu- und Abführleitungen erfindungsgemäß derart ausgebildet, dass sie zum Zu- und Abführen von Spülflüssigkeit zu und von dem Element geeignet sind. Die Spülflüssigkeiten sind mittels einer zweiten spülflüssigkeitsführenden Anschlussleitung in die gasführbare Zuführleitung einspeisbar und dienen zum Spülen des gesamten Leitungssystems, um das Verkleben und Verstopfen, insbesondere der Abführleitung, durch Inhaltsstoffe der Substanzen der Probe zu vermeiden. Die Spülflüssigkeit ist eine sterile Flüssigkeit. Nach dem Spülvorgang wird das Leitungssystem wiederum mit Gas aus der ersten Anschlussleitung mit geringem Überdrück entleert. Hierbei handelt es sich vorzugsweise um ein steriles Gas. Ein derartiger Spülvorgang kann optional zwischen zwei Probenahmevorgängen durchgeführt werden. Vorteilhaft weist ein Verfahren zur Entnahme von flüssigen oder gasförmigen Proben aus dem mit einem Medium gefüllten ersten Behälter und/oder Rohren mittels Unterdruck folgende Schritte auf:
- Zuführen eines Gases zu dem als Rückstellventil wirkenden Element auf der dem in dem ersten Behälter angeordneten Medium abgewandten Seite des Elements mittels einer mit mindestens einem Ventil gegenüber anderen Leitungen sperrbaren Zuführleitung,
- Abführen des Gases von dem als Rückstellventil wirkenden Element mittels einer Abführleitung und Öffnen einer in der Abführleitung angeordneten als Schließventil wirkenden Vorrichtung solange, bis die Zu- und Abführleitungen probenfrei sind,
- Schließen mindestens eines Ventils zum Abkoppeln der Zuführleitung von einem Gasversorgungsanschluss,
- Erzeugung eines Unterdrucks in der Abführleitung, beispielsweise mittels einer Pumpeinrichtung,
- selbsttätiges sich Öffnen des Elements mittels des erzeugten Unterdrucks und Fördern einer zu entnehmenden Probe aus dem Behälter in die Abführleitung,
- Zuführen eines mit Überdruck erneut zugeführten Gases,
- selbsttätiges sich Schließen des Elements mittels des erzeugten Überdrucks, und
- Beförderung der Probe aus der Abführleitung mittels des mit Überdruck erneut zugeführten Gases.

Zusätzlich kann zur Vermeidung von Verstopfungen und Verklebungen der Abführleitung das Verfahren den Schritt der Zuführung von Spülflüssigkeit und die Durchführung des Integritätstests die Zuführung von Gas unter Abschluss des Vorrichtungsinnenraums mittels Ventilen gegenüber den umliegenden Systemen beinhalten.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: eine schematische Darstellung einer erfindungsgemäßen Vorrichtung zur Entnahme von Proben,
- Fig. 2: eine schematische Darstellung eines Ausschnitts der Vorrichtung zur Ent- nahme von Proben gemäß einer Ausführungsform der Erfindung, und
- Fig. 3: eine schematische Darstellung eines Ausschnitts der Vorrichtung zur Ent- nahme von Proben gemäß einer zweiten Ausführungsform der Erfindung.

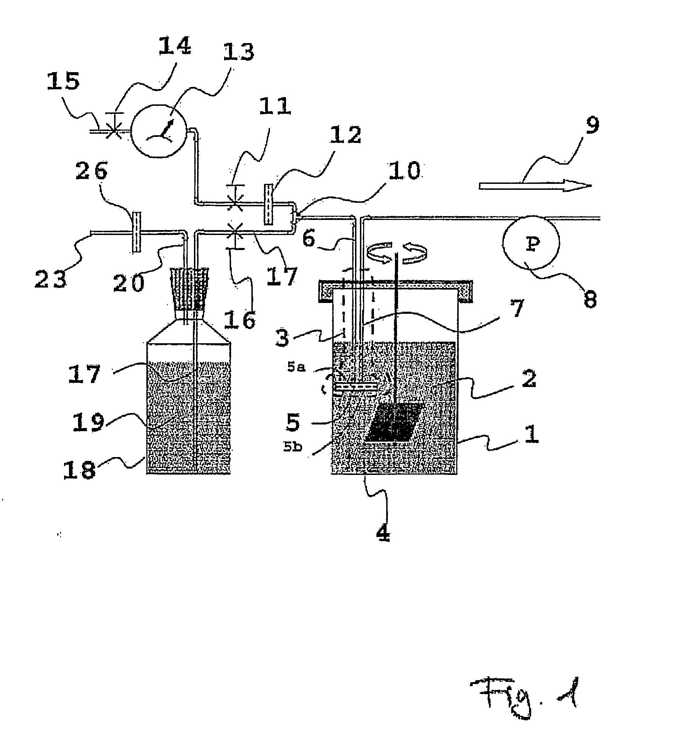

Fig. 1 zeigt in einer schematischen Darstellung eine erfindungsgemäße Vorrichtung zur Entnahme von Proben aus einem mit einem Medium gefüllten ersten Behälter 1. In das in dem Behälter 1 enthaltene Medium 2 ist eine Probenahmesonde 3 und ein Rührer 4 zum Umrühren des Mediums 2 eingeführt. Innerhalb der Probenahmesonde 3 ist ein in dieser Figur nicht näher dargestelltes als Rückstellventil wirkendes Element 5 beispielsweise aus Metall oder Kunststoff angeordnet. Das Element 5 weist eine sterile Rückgrenzseite 5a und eine zu dem Medium hingewandte Vordergrenzseite 5b auf, wobei das Medium steril oder nicht-steril ausgebildet sein kann.

An der Rückgrenzseite 5a des Elementes 5 - also auf der sterilen Grenzseite des als Rückstellventil wirkenden Elementes 5 - sind eine gasführbare Zuführleitung und eine probeführbare Abführleitung 7 angeordnet. Über die Abführleitung 7 kann eine Probe, die durch einen Unterdruck einer Pumpe 8 durch das als Rückstellventil 5 hindurch, welches den Eingang zu einer Leitungsanordnung, bestehend aus Zu- und Abführleitung, bildet, die mit einem zweiten in dieser Figur nicht dargestellten Behälter verbunden sein können, aus dem Medium angesaugt wird, in eine hier nicht gezeigte Detektoreinrichtung oder einen hier nicht direkt gezeigten Vorratsbehälter abgeführt werden, wie es durch den Pfeil 9 angedeutet wird.

Die Zuführleitung 6 ist mittels eines T-Stückes 10 mit einer ersten gasführenden Anschlussleitung verbunden, die wiederum mit einem Gasversorgungsanschluss 15 in Verbindung steht. Über den Gasversorgungsanschluss 15 wird Druckluft der Zuführleitung 6 zugeführt, sofern sperrbare Ventile 11 und 14 in ihrer Öffnungsstellung angeordnet sind.

Um eine Zuführung von steriler Druckluft sicherzustellen, ist ein Sterilfilter 12 angeordnet. Zusätzlich wird die unter leichtem Überdruck zugeführte Druckluft mittels eines Manometers 13 druckgeprüft.

An dem weiteren Ende des T-Stückes 10 ist eine zweite spülflüssigkeitsführende Anschlussleitung 17 angeschlossen, die mit einem Behälter 18 in Verbindung steht, in dem Spülflüssigkeit 19, wie beispielsweise destilliertes Wasser, in ausreichendem Maße vorhanden ist. Eine Gas- und Flüssigkeitsanschlussleitung 20 verbindet den Behälter 18 über einen darin angeordneten zweiten Sterilfilter 26 mit einem weiteren Gasversorgungsanschluss 23, um einen Druckausgleich für den Behälter 18 zu schaffen. Dieser Gasversorgungsanschluss 23 ist wahlweise zuschaltbar.

Sofern ein Spülvorgang durchgeführt werden soll, wird ein Ventil 16 zur Zuführleitung 6 hin geöffnet, während die Ventile 11 und 14 der ersten gasführenden Anschlussleitung geschlossen werden. Nachdem die Spülflüssigkeit die Zu- und Abführleitungen 6, 7 über die Pumpe 8 verlassen hat, und eine Spülung der Leitungen 6, 7 und der Rückgrenzseite 5a des Elementes 5 durchgeführt worden ist, wird das Ventil 16 geschlossen und die ersten und zweiten Ventile 11, 14 werden geöffnet, um einen Entleerungsvorgang des Leitungssystems mit den Zu- und Abführleitungen 6, 7 mittels der zugeführten sterilen Druckluft zu bewirken.

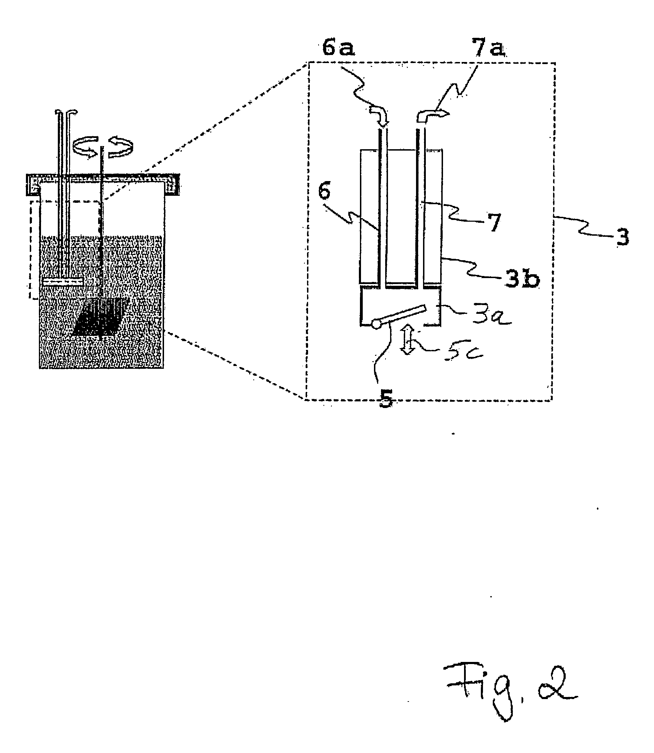

Fig. 2 zeigt in einer schematischen Darstellung einen Ausschnitt der Vorrichtung zur Entnahme von Proben gemäß einer Ausführungsform der Erfindung. In dieser Darstellung ist die Probenahmesonde 3 vergrößert dargestellt. Wie der vergrößerten Darstellung der Probenahmesonde entnehmbar ist, besteht diese aus dem unterseitigen zweiten Behälter 3a, der Zuführleitung 6 und der Abführleitung 7, deren Fließrichtungen durch die Pfeile 6a und 7a angedeutet werden, und einer Ummantelung 3b, die wärmeisolierend wirkt. Eine derartige Ummantelung 3b kann zusätzlich mit einer hier nicht gezeigten Heiz- oder Kühleinrichtung verbunden sein, um eine Temperierung oder Kühlung der Probe während ihres Entnahmevorganges zur Verlängerung ihrer Aussagekraft zu erreichen.

Bei einem Probenahmevorgang findet folgender Ablauf statt:
Die Pumpe 8 erzeugt, während das erste und zweite Ventil 11, 14 geschlossen sind, einen Unterdruck innerhalb des Leitungssystems, der eine Probe aus dem zu untersuchenden Medium 2 durch das geöffnete als Rückstellventil wirkende Element 5 hindurch zunächst in den zweiten Behälter 3a befördert. Der erzeugte Unterdruck kann ein automatisches sich Öffnen des Elementes 5 bewirken, dessen Öffnungs- und Schließbewegung durch den Pfeil 5c angedeutet wird.

Das gewünschte Probevolumen der zu entnehmenden Probe wird mittels der Pumpstärke der Pumpe 8 reguliert. Nach Erreichen eines vorbestimmten Probevolumens mit einer vorbestimmten Förderzeit wird Druckluft mit einem leichten Überdruck über die Zuführleitung 6 zugeführt, woraufhin ein automatisches Sichschließen des Elementes 5 stattfindet. Zeitgleich wird die Probe mittels des Druckluftüberdruckes bei geschlossenem Ventil 16 und offenen Ventilen 11 und 14 aus dem Leitungssystem mit den Leitungen 6, 7 derart gefördert, dass es über die Abführleitung 7 in einen hier nicht gezeigten Vorratsbehälter eingespeist wird. Gleichzeitig bewirkt die zugeführte Druckluft zuverlässig eine Entleerung des Leitungssystems, so dass dieses probenfrei und bei Verwendung von steriler Druckluft steril ist.

Für einen durchführbaren Integritätstest zur Überprüfung der Funktion der Probenahmevorrichtung wird über den Gasversorgungsanschluss 15 bei geschlossenem Ventil 16 und nicht betriebener Pumpe 8 Druckluft zur Erzeugung eines Überdrucks dem Leitungssystem zugeführt. Der Überdruck bleibt nach Schließen des Ventils 14 weiterhin erhalten, sofern das als Rückstellventil wirkende Element 5, welches eine Art Sterilgrenze zu dem restlichen Medium 2 bildet, dicht ist. Wenn der Druck hingegen abfällt, deutet dies auf ein undichtes Element 5 hin.

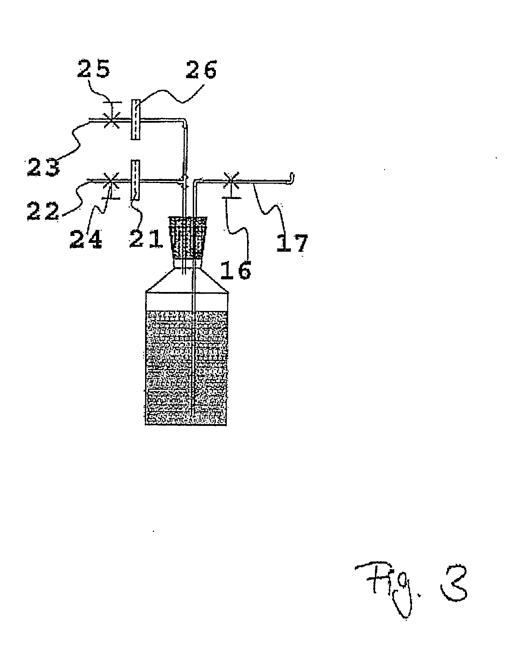

Fig. 3 zeigt in einer schematischen Darstellung einen Ausschnitt der Vorrichtung zur Entnahme von Proben gemäß der Erfindung. Es ist zusätzlich ein Spülflüssigkeitsversorgungsanschluss 22 mit dem Behälter 18 über einen spülflüssigkeitsführenden Teil der Leitung 20 und einem jeweils dazwischen angeordnetem weiteren Sterilfilter 21 und weiterem Ventil 24 verbunden. Der Spülflüssigkeitsversorgungsanschluss 22 dient zum Wiederauffüllen des Behälters 18 mit Spülflüssigkeit. Der gasführende Teil der Leitung 20 weist zusätzlich ein weiteres Ventil 25 zwischen dem Filter 26 und dem Gasversorgungsanschluss 23 zum Zuführen, Abführen oder Dosieren von Gas auf.

Die Ausführung der Erfindung ist nicht nur auf das beschriebene Beispiel und die hervorgehobenen Aspekte beschränkt, sondern ist im Rahmen der Ansprüche ebenso in einer Vielzahl von Abwandlungen möglich, die im Rahmen fachgemäßen Handelns liegen.

### Bezugszeichenliste

- 1: erster Behälter
- 2: Medium
- 3: Probenahmesonde
- 3a: zweiter Behälter
- 3b: wärmeisolierende Ummantelung
- 4: Rührer
- 5: als Rückstellventil wirkendes Element
- 5a: Rückseite des Elementes
- 5b: Vorderseite des Elementes
- 5c: Bewegungsrichtung des Elementes
- 6: Zuführleitung
- 6a: Fließrichtung innerhalb der Zuführleitung
- 7: Abführleitung
- 7a: Fließrichtung innerhalb der Abführleitung
- 8: Pumpe
- 9: Abführrichtung
- 10: T-Stück
- 11, 14, 16, 24, 25: Ventile
- 12, 21, 26: Sterilfilter
- 13: Manometer
- 25,23: Gasversorgungsanschuss
- 17: spülflüssigkeitsführende Anschlussleitung
- 18: dritter Behälter
- 19 .: Spülflüssigkeit
- 20: Gas- und Spülflüssigkeitsanschlussleitung
- 22: Spülflüssigkeitsversorgungsanschluss
- 23: Gasversorgungsanschluss

## Patentansprüche

1. Vorrichtung zur Entnahme von flüssigen oder gasförmigen Proben aus mit einem Medium (2) gefüllten ersten Behältern (1) und/oder Rohren, insbesondere aus Fermentern, mittels Unterdruck, wobei innerhalb einer Probesonde (3) ein als Rückstellventil wirkendes Element (5) als Eingang für die zu entnehmende Probe angeordnet ist, wobei auf der dem in dem ersten Behälter (1) angeordnetem Medium (2) abgewandten Seite des Elements (5) eine gasführbare Zuführleitung (6) und eine probeabführbare Abführleitung (7) angeordnet sind,
**dadurch gekennzeichnet, dass**
das als Rückstellventil wirkende Element (5) derart ausgebildet ist, dass es mittels dem Zuführen eines Gases mit Überdruck selbsttätig schließbar ist und die Abführleitung (7) mit einer den Unterdruck erzeugenden Einrichtung (8) verbunden ist, wobei die gasführbare Zuführleitung (6) an eine erste gasführende Anschlussleitung zum Verbinden der Zuführleitung (6) mit einem Gasversorgungsanschluss (15) angeschlossen ist und die Zuführleitung (6) an eine zweite spülflüssigkeitführende Anschlussleitung (17) angeschlossen ist.

2. Vorrichtung nach Anspruch 1,
**dadurch gekennzeichnet, dass**
die gasführbare Zuführleitung (6) und die probeabführbare Abführleitung (7) jeweils mit einem probeaufnehmenden zweiten Behälter (3a) verbunden sind.

3. Vorrichtung nach Anspruch 1,
**dadurch gekennzeichnet, dass**
das Element (5) innerhalb einer Leitungsanordnung zwischen Zuführ- und Abführleitung (6, 7) angeordnet ist.

4. Vorrichtung nach einem der Ansprüche 1 - 3,
**dadurch gekennzeichnet, dass**
die gasführbare Zuführleitung (6) und die Abführleitung (7) derart ausgebildet sind, dass sie zum Zu- und Abführen von Gasen mit Überdruck zu und von dem als Rückstellventil wirkenden Element (5) geeignet sind.

5. Vorrichtung nach einem der vorangegangenen Ansprüche,
**dadurch gekennzeichnet, dass**
die Zuführleitung (6) und die Abführleitung (7) innerhalb des ersten Behälters (1) angeordnet sind und zumindest teilweise eine Ummantelung (3b) zur Temperierung und/oder Kühlung der Leitungen (6, 7) aufweisen.

6. Vorrichtung nach Anspruch 5,
**gekennzeichnet durch**
eine Heizeinrichtung zum Temperieren oder eine Kühleinrichtung zum Kühlen der Zuführleitung (6) und der Abführleitung (7) innerhalb der Ummantelung (3b).

7. Vorrichtung nach einen der vorangegangenen Ansprüche,
**dadurch gekennzeichnet, dass**
ein erstes und zweites Ventil (11, 14) im Bereich des ersten und zweiten Endes der Anschlussleitung angeordnet sind.

8. Vorrichtung nach Anspruch 7,
**dadurch gekennzeichnet, dass**
ein Drucksensor (13) in der gasführenden Anschlussleitung angeordnet ist.

9. Vorrichtung nach einem der Ansprüche 4 - 8,
**dadurch gekennzeichnet, dass**
ein erster Sterilfilter (12) in der gasführenden Anschlussleitung angeordnet ist.

10. Vorrichtung nach einem der vorangegangenen Ansprüche,
**dadurch gekennzeichnet, dass**
die Zu- und Abführleitungen (6, 7) derart ausgebildet sind, dass sie zum Zu- und Abführen von Spülflüssigkeiten zu und von dem Element (5) geeignet sind.

11. Vorrichtung nach einem der vorangegangenen Ansprüche,
**dadurch gekennzeichnet, dass**
die spülftüssigkeitführende Anschlussleitung (17) mit einem eine Spülflüssigkeit (19) enthaltenden dritten Behälter (18) verbunden ist.

12. Vorrichtung nach Anspruch 11,
**dadurch gekennzeichnet, dass**
der dritte Behälter (18) zum Druckausgleich mit einem weiteren Gasversorgungsanschluss (23) über eine weitere gasführende Anschlussleitung (20) mit einem darin angeordneten weiteren Sterilfilter (26) verbunden ist.

13. Vorrichtung nach einem der Ansprüche 10 - 12,
**dadurch gekennzeichnet, dass**
der dritte Behälter (18) zusätzlich mit einem Spülflüssigkeitsversorgungsanschluss (22) über eine weitere spülflüssigkeitszuführende Anschlussleitung (20) mit einem darin angeordneten weiteren Sterilfilter (21) verbunden ist.

14. Verfahren zur Entnahme von flüssigen oder gasförmigen Proben aus mit einem Medium (2) gefüllten Behältern (1) und/oder Rohren, insbesondere aus Fermentern mittels Unterdruck, wobei innerhalb einer Probesonde (3) ein als Rückstellventil wirkendes Element (5) als Eingang für die zu entnehmende Probe angeordnet ist, wobei auf der dem in dem ersten Behälter (1) angeordnetern Medium (2) abgewandten Seite des Elements (5) eine gasführbare Zuführleitung (6) und eine probeabführbare Abführleitung (7) angeordnet sind,
**gekennzeichnet durch**
folgende Schritte:
- Zuführen eines Gases mit Überdruck zu dem als Rückstellventil wirkenden Element (5), welches selbsttätig schließbar ist, auf der dem in dem Behälter (1) angeordneten Medium (2) abgewandten Seite des Elements (5) mittels einer gegenüber anderen Leitungen sperrbaren und an eine zweite spülflüssigkeitführende Anschlussleitung (17) angeschlossenen Zuführleitung (6),
- Abführen des Gases von dem als Rückstellventil wirkenden Element (5) mittels einer Abführleitung (7) und Öffnen einer in der Abführleitung (7) angeordneten als Schließventil wirkenden Vorrichtung (8) solange, bis die Zu- und Abführleitungen (6, 7) probenfrei sind,
- Schließen mindestens eines Ventils (11) zum Abkoppeln der Zuführleitung (6) von einem Gasversorgungsanschluss (15),
- Erzeugung eines Unterdrucks in Bezug auf den im Behälter (1) bestehenden Druck in der Abführleitung (7) mittels einer mit der Abführleitung verbundenen den Unterdruck erzeugenden Einrichtung,
- selbsttätiges Sichöffnen des Elements (5) mittels des erzeugten Unterdrucks und Fördern einer zu entnehmenden Probe aus dem Behälter (1) in die Abführleitung (7),
- Zuführen eines mit Überdruck in Bezug auf den im Behälter (1) bestehenden Druck erneut zugeführten Gases,
- selbsttätiges Sichschließen des Elements (5) mittels des erzeugten Überdrucks, und
- Beförderung der Probe aus der Abführleitung (7) mittels des mit Überdruck erneut zugeführten Gases.

15. Verfahren nach Anspruch 14,
**dadurch gekennzeichnet, dass**
zur Vermeidung von durch Inhaltsstoffe der Probe verursachte Verstopfungen und Verklebungen innerhalb der Abführleitung (7) eine Spülflüssigkeit (19) nach dem Schritt der Beförderung der Probe aus der Abführleitung (7) über die Zuführleitung (6) zugeführt und über die Abführleitung (7) abgeführt wird.

16. Verfahren nach Anspruch 15,
**dadurch gekennzeichnet, dass**
nach dem Schritt des Zu- und Abführens der Spülflüssigkeit (19) die Schritte des Zu- und Abführens des Gases wiederholt werden.

## Claims

1. Device for taking liquid or gaseous samples from first containers (1) and/or tubes which are filled with a medium (2), in particular from fermenters, by means of negative pressure, wherein an element (5) which acts as a non-return valve is disposed within a sample probe (3) as an inlet for the sample which is to be taken, wherein a supply line (6) which is able to convey gas and a discharge line (7) which is able to discharge samples are disposed on the side of the element (5) which is remote from the medium (2) disposed in the first container (1),
**characterised in that**
the element (5) which acts as a non-return valve is formed in such a way that it can be automatically closed by means of supplying a gas at positive pressure and the discharge line (7) is connected to a device (8) generating the negative pressure, wherein the supply line (6) which is able to convey gas is connected to a first gas-conveying connecting line for connecting the supply line (6) to a gas supply connection (15) and the supply line (6) is connected to a second connecting line (17) which conveys rinsing fluid.

2. Device according to Claim 1,
**characterised in that**
the supply line (6) which is able to convey gas and the discharge line (7) which is able to discharge samples are in each case connected to a second container (3a) which receives the samples.

3. Device according to Claim 1,
**characterised in that**
the element (5) is disposed within a line arrangement between the supply and the discharge line (6, 7).

4. Device according to any one of Claims 1 - 3,
**characterised in that**
the supply line (6) which is able to convey gas and the discharge line (7) are formed in such a way that they are suitable for supplying and discharging gases at positive pressure to and from the element (5) which acts as a non-return valve.

5. Device according to any one of the preceding Claims,
**characterised in that**
the supply line (6) and the discharge line (7) are disposed within the first container (1) and have a at least in part sheathing (3b) for temperature control and/or cooling of the lines (6, 7).

6. Device according to Claim 5,
**characterised by**
a heating device for temperature control or a cooling device for cooling of the supply line (6) and the discharge line (7) within the sheathing (3b).

7. Device according to any one of the preceding Claims,
**characterised in that**
a first and a second valve (11, 14) are disposed in the region of the first and the second end of the connecting line.

8. Device according to Claim 7,
**characterised in that**
a pressure sensor (13) is disposed in the gas-conveying connecting line.

9. Device according to any one of Claims 4 - 8,
**characterised in that**
a first sterile filter (12) is disposed in the gas-conveying connecting line.

10. Device according to any one of the preceding Claims,
**characterised in that**
the supply and discharge lines (6, 7) are formed in such a way that they are suitable for supplying and discharging rinsing fluids to and from the element (5).

11. Device according to any one of the preceding Claims,
**characterised in that**
the connecting line (17) which conveys rinsing fluids is connected to a third container (18) which contains a rinsing fluid (19).

12. Device according to Claim 11,
**characterised in that,**
for the purpose of pressure equalisation, the third container (18) is connected to a further gas supply connection (23) via a further gas-conveying connecting line (20) with a further sterile filter (26) disposed therein.

13. Device according to any one of Claims 10 - 12,
**characterised in that**
the third container (18) is additionally connected to a rinsing fluid supply connection (22) via a further connecting line (20) which supplies rinsing fluid and has a further sterile filter (21) disposed therein.

14. Method for taking liquid or gaseous samples from containers (1) and/or tubes which are filled with a medium (2), in particular from fermenters, by means of negative pressure, wherein an element (5) which acts as a non-return valve is disposed within a sample probe (3) as an inlet for the sample which is to be taken, wherein a supply line (6) which is able to convey gas and a discharge line (7) which is able to discharge samples are disposed on the side of the element (5) which is remote from the medium (2) disposed in the first container (1),
**characterised by**
the following steps:
- supplying a gas at positive pressure to the element (5) which acts as a non-return valve and which can be automatically closed on the side of the element (5) which is remote from the medium (2) disposed in the container (1) by means of a supply line (6) which can be shut off from other lines and is connected to a second connecting line (17) which conveys rinsing fluid,
- discharging the gas from the element (5) which acts as a non-return valve by means of a discharge line (7) and opening a device (8) which is disposed in the discharge line (7) and acts as a shut-off valve until the supply and discharge lines (6, 7) are sample-free,
- closing at least one valve (11) in order to disconnect the supply line (6) from a gas supply connection (15),
- generating in the discharge line (7) a negative pressure with respect to the pressure prevailing in the container (1) by means of a device which is connected to the discharge line and generates the negative pressure,
- automatically opening the element (5) by means of the generated negative pressure and conveying a sample which is to be taken out of the container (1) into the discharge line (7),
- supplying a gas which is again supplied at positive pressure with respect to the pressure prevailing in the container (1),
- automatically closing the element (5) by means of the generated positive pressure, and
- conveying the sample out of the discharge line (7) by means of the gas again supplied at positive pressure.

15. Method according to Claim 14,
**characterised in that,**
in order to prevent blockages and adhesions within the discharge line (7) which are caused by constituents of the sample, a rinsing fluid (19) is supplied via the supply line (6) and discharged via the discharge line (7) after the step of conveying the sample out of the discharge line (7).

16. Method according to Claim 15,
**characterised in that** the steps of supplying and discharging the gas are repeated after the step of supplying the rinsing fluid (19).

## Revendications

1. Dispositif pour le prélèvement d'échantillons liquides ou gazeux hors de premiers récipients (1) et/ou tubes, remplis d'un médium (2), en particulier hors de fermenteurs, au moyen d'une dépression, sachant qu'à l'intérieur d'une sonde de prélèvement d'échantillons (3) est disposé un élément (5) agissant comme une soupape de rappel et formant une entrée pour l'échantillon à prélever, une conduite d'admission (6) apte à acheminer le gaz et une conduite d'évacuation (7) apte à évacuer les échantillons étant disposées sur le côté de l'élément (5), détourné du médium (2) contenu dans le premier récipient (1),
**caractérisé en ce que** l'élément (5) agissant comme une soupape de rappel est réalisé de telle sorte qu'il peut se fermer automatiquement au moyen de l'admission d'un gaz avec une surpression, et la conduite d'évacuation (7) est reliée à un dispositif (8) générant la dépression, la conduite d'admission (6) apte à acheminer le gaz étant raccordée à une première conduite de raccordement acheminant le gaz, afin de relier la conduite d'admission (6) à un raccord d'alimentation en gaz (15), et la conduite d'admission (6) étant raccordée à une deuxième conduite de raccordement (17) acheminant un liquide de lavage.

2. Dispositif selon la revendication 1, **caractérisé en ce que** la conduite d'admission (6) apte à acheminer le gaz et la conduite d'évacuation (7) apte à évacuer les échantillons est reliée, chacune, à un deuxième récipient (3a) recevant les échantillons.

3. Dispositif selon la revendication 1, **caractérisé en ce que** l'élément (5) est disposé à l'intérieur d'un système de conduite entre la conduite d'admission (6) et la conduite d'évacuation (7).

4. Dispositif selon l'une quelconque des revendications 1 à 3, **caractérisé en ce que** la conduite d'admission (6) apte à acheminer le gaz et la conduite d'évacuation (7) sont réalisées de telle sorte qu'elles sont aptes à acheminer des gaz avec une surpression vers l'élément (5) agissant comme une soupape de rappel et à évacuer les gaz avec une surpression à partir dudit élément.

5. Dispositif selon l'une quelconque des revendications précédentes, **caractérisé en ce que** la conduite d'admission (6) et la conduite d'évacuation (7) sont disposées à l'intérieur du premier récipient (1) et comportent au moins partiellement une gaine (3b) pour tempérer et/ou refroidir les conduites (6. 7).

6. Dispositif selon la revendication 5, **caractérisé par** un dispositif de chauffage pour tempérer ou un dispositif de refroidissement pour refroidir la conduite d'admission (6) et la conduite d'évacuation (7) à l'intérieur de la gaine (3b).

7. Dispositif selon l'une quelconque des revendications précédentes, **caractérisé en ce qu'**une première et une deuxième vanne (11, 14) sont disposées dans la zone de la première et de la deuxième extrémité de la conduite de raccordement.

8. Dispositif selon la revendication 7, **caractérisé en ce qu'**un capteur de pression (13) est disposé dans la conduite de raccordement acheminant le gaz.

9. Dispositif selon l'une quelconque des revendications 4 à 8, **caractérisé en ce qu'**un premier filtre stérile (12) est disposé dans la conduite de raccordement acheminant le gaz.

10. Dispositif selon l'une quelconque des revendications précédentes, **caractérisé en ce que** la conduite d'admission (6) et la conduite d'évacuation (7) sont réalisées de telle sorte qu'elles sont aptes à acheminer des liquides de lavage vers l'élément (5) et à évacuer des liquides de lavage depuis ledit élément.

11. Dispositif selon l'une quelconque des revendications précédentes, **caractérisé en ce que** la conduite de raccordement (17) acheminant le liquide de lavage est reliée à un troisième récipient (18) contenant un liquide de lavage (19).

12. Dispositif selon la revendication 11, **caractérisé en ce que** le troisième récipient (18), en vue de l'équilibrage de la pression avec un autre raccord d'alimentation en gaz (23) est relié via une autre conduite de raccordement (20) acheminant le gaz à un autre filtre stérile (26) disposé dans cette dernière.

13. Dispositif selon l'une quelconque des revendications 10 à 12, **caractérisé en ce que** le troisième récipient (18) est relié, en plus, à un raccord d'alimentation en liquide de lavage (22) via une autre conduite de raccordement (20) acheminant le liquide de lavage avec un autre filtre stérile (21) disposé dans cette dernière.

14. Procédé pour le prélèvement d'échantillons liquides ou gazeux hors de premiers récipients (1) et/ou tubes, remplis d'un médium (2), en particulier hors de fermenteurs, au moyen d'une dépression, sachant qu'à l'intérieur d'une sonde de prélèvement d'échantillons (3) est disposé un élément (5) agissant comme une soupape de rappel et formant une entrée pour l'échantillon à prélever, une conduite d'admission (6) apte à acheminer le gaz et une conduite d'évacuation (7) apte à évacuer les échantillons étant disposées sur le côté de l'élément (5), détourné du médium (2) contenu dans le premier récipient (1),
**caractérisé par** les étapes suivantes :
- acheminement d'un gaz avec une surpression vers l'élément (5), agissant comme une soupape de rappel et apte à se fermer automatiquement, sur le côté de l'élément (5), détourné du fluide (2) contenu dans le premier récipient (1), au moyen d'une conduite d'admission (6), apte à être fermée par rapport aux autres conduites et raccordée à une deuxième conduite de raccordement (17) acheminant le liquide de lavage,
- évacuation du gaz depuis l'élément (5), agissant comme une soupape de rappel, au moyen d'une conduite d'évacuation (7), et ouverture d'un dispositif (8), disposé dans la conduite d'évacuation (7) et agissant comme une vanne de fermeture, jusqu'à ce que la conduite d'admission (6) et la conduite d'évacuation (7) ne contiennent plus d'échantillons,
- fermeture d'au moins une vanne (11) pour déconnecter la conduite d'admission (6) d'un raccord d'alimentation en gaz (15),
- production d'une dépression, par rapport à la pression régnant à l'intérieur du récipient (1), dans la conduite d'évacuation (7) au moyen d'un dispositif relié à la conduite d'évacuation et générant la dépression,
- ouverture automatique de l'élément (5) au moyen de la dépression générée et transport d'un échantillon à prélever, hors du récipient (1) dans la conduite d'évacuation (7).
- admission d'un gaz acheminé à nouveau avec une surpression par rapport à la pression régnant à l'intérieur du récipient (1),
- fermeture automatique de l'élément (5) au moyen de la surpression générée, et
- transport de l'échantillon hors de la conduite d'évacuation (7) au moyen du gaz acheminé à nouveau avec la surpression.

15. Procédé selon la revendication 14, **caractérisé en ce que**, pour éviter des engorgements et des agglutinations, dus aux ingrédients de l'échantillon, à l'intérieur de la conduite d'évacuation (7), un liquide de lavage (19) est acheminé via la conduite d'admission (6) et est évacué via la conduite d'évacuation (7), après l'étape de transport de l'échantillon hors de la conduite d'évacuation (7).

16. Procédé selon la revendication 15, **caractérisé en ce que** les étapes d'admission et d'évacuation du gaz sont répétées après l'étape de l'admission et de l'évacuation du liquide de lavage (19).
